**Europäisches Patentamt**

**European Patent Office**

**Office européen des brevets**

(19)

(11) Numéro de publication : **0 410 877 B1**

(12)

# FASCICULE DE BREVET EUROPEEN

(45) Date de publication du fascicule du brevet :
**22.12.93 Bulletin 93/51**

(51) Int. Cl.⁵ : **C02F 3/34,** C02F 3/10,
C02F 1/28

(21) Numéro de dépôt : **90402135.9**

(22) Date de dépôt : **25.07.90**

(54) **Composition de matière pour l'épuration chimique et biologique des eaux polluées et procédé de préparation de cette composition.**

Le dossier contient des informations
techniques présentées postérieurement au
dépôt de la demande et ne figurant pas dans le
présent fascicule.

(30) Priorité : **28.07.89 FR 8910226**

(43) Date de publication de la demande :
**30.01.91 Bulletin 91/05**

(45) Mention de la délivrance du brevet :
**22.12.93 Bulletin 93/51**

(84) Etats contractants désignés :
**AT BE CH DE ES GB GR IT LI LU NL SE**

(56) Documents cités :
**EP-A- 0 077 856**
**WO-A-86/07346**
**FR-A- 1 314 049**
**FR-A- 2 481 257**
**US-A- 4 200 528**

(73) Titulaire : **Van den Hecke, Jean-Claude**
**3 rue de la Gare, Vilbert**
**F-77540 Rosay en Brie (FR)**
Titulaire : **Dubourg, Estelle**
**33 Avenue de la Gare**
**F-91160 Longjumeau (FR)**

(72) Inventeur : **Van den Hecke, Jean-Claude**
**3 rue de la Gare, Vilbert**
**F-77540 Rosay en Brie (FR)**
Inventeur : **Dubourg, Estelle**
**33 Avenue de la Gare**
**F-91160 Longjumeau (FR)**

(74) Mandataire : **Bouju, André et al**
**Cabinet Bouju Derambure (Bugnion) S.A. B.P.**
**6250**
**F-75818 Paris Cédex 17 (FR)**

Jouve, 18, rue Saint-Denis, 75001 PARIS

## Description

La présente invention concerne une composition de matière pour l'épuration chimique et biologique des eaux polluées.

L'invention vise en particulier une composition minérale et biologique destinée à être répandue dans l'eau à épurer, permettant notamment l'épuration et la dégradation des rejets fermentescibles urbains, industriels, agro-alimentaires, solides ou liquides, les pollutions accidentelles, l'entrophysation et la destrophysation des rivières, des étangs et la prévention des pollutions ci-dessus.

On sait que certaines bactéries sont capables d'épurer les eaux polluées, notamment par des rejets industriels.

L'efficacité de ces bactéries peut être augmentée en faisant réagir celles-ci avec un agent mutagène permettant d'accroître notamment la production d'enzymes de ces bactéries. Toutefois, les bactéries mutées obtenues de cette façon ne sont pas stables et reviennent rapidement à leur stade initial.

Ainsi, pour maintenir l'efficacité de ces bactéries vis-à-vis du milieu à épurer, il est nécessaire d'additionner en continu à ce milieu, des bactéries préalablement mutées.

Il en résulte que ce procédé d'épuration par bactéries mutées est un procédé très onéreux, de sorte qu'il n'est pas adapté à un traitement à grande échelle sur de grandes étendues d'eau polluée.

Le but de la présente invention est de remédier aux inconvénients des méthodes d'épuration biologiques connues en proposant une composition de matière à la fois efficace et peu onéreuse.

Suivant l'invention, cette composition de matière, destinée à être répandue dans l'eau à épurer est caractérisée en ce qu'elle comprend au moins les deux matières minérales suivantes, sous forme granulaire :
- un carbonate de calcium poreux, riche en oligo-éléments,
- un silicate d'alumine hydraté d'origine zéolitique renfermant des métaux alcalino-terreux et présentant des microcanaux,

ces deux matières renfermant à l'état adsorbé des bactéries spécifiques pour la dégradation biologique des matières organiques à chaîne carbonée.

La première matière, c'est-à-dire le carbonate de chaux, présente une grande porosité et donc une grande surface de contact favorable aux échanges ioniques avec le milieu à épurer.

Cette matière est capable :
- de transformer le phosphate soluble contenu dans le milieu pollué en phosphate tricalcique insoluble,
- d'abaisser la teneur en fluor et en magnésium du milieu pollué,
- d'agir sur la dureté de l'eau et la radioactivité de celle-ci,
- d'adsorber une grande quantité de bactéries dont l'activité biologique aura un pouvoir tampon important (pH) tout en dégradant la matière organique, notamment les hydrocarbures contenus dans l'eau polluée, et
- de participer ainsi à l'équilibre phytoplancton-zooplancton, au profit de ce dernier.

La deuxième matière, constituée par un silicate d'alumine hydraté permet de piéger les ions ammonium contenus dans l'eau polluée qui sont transformés en molécules inoffensives par les bactéries adsorbées dans la matière.

Comme le premier matériau, le deuxième matériau ci-dessus constitue un véritable abri pour les bactéries. Ce deuxième matériau permet également d'adsorber par échange ionique certains métaux lourds contenus dans le milieu pollué.

De préférence, la première matière est constituée par du lithothmamnium calcareum. Cette matière est d'origine végétale planctonique sécrétée par des algues mono-cellulaires. Sa densité apparente est de 1,05 à 1,08, et sa densité réelle égale à 2,7.

L'avantage du lithothmamnium calcareum (corallinacée), par rapport aux autres carbonates de chaux, est une grande porosité qui augmente considérablement la surface de contact et les échanges ioniques.

Au microscope, le lithothamnium décèle des cavités de 15 à 20 microns de long sur 8 à 10 microns de large, où se loge un protoplaste qui se contracte lors du séchage et laisse ainsi une place vide pour l'activité biologique et la réserve d'oxygène.

Ce produit est prélevé dans des fonds marins de 20 à 50 mètres.

Il existe également un carbonate de calcium de qualité sensiblement égale, extraite des carrières du Nord Est de Paris, appelée "craie".

La deuxième matière est une pierre volcanique de la famille des zéolites (silicate d'alumine hydraté alcalino terreux) formé en des conditions de température et pression bien définies et en présence d'eau.

Cette matière est de structure caverneuse formant des micro-canaux dont le diamètre est de l'ordre de quelques Å qui peuvent représenter jusqu'à 50% du volume ; ils sont remplis d'eau évacuable à la chaleur selon un processus réversible jusqu'à 350°C.

La structure renferme des cations alcalino-terreux de compensation électrique (déséquilibres de charge des unités Si-Al) qui sont situés soit dans les micro-canaux, soit dans des emplacements adjacents aux cavités. Ils sont en général échangeables avec des métaux lourds contenus dans le milieu pollué.

Les propriétés de cette matière sont avant tout : la capacité d'échanges ioniques, sa grande surface d'absorption qui est de plusieurs centaines de m²/g lui permettant ainsi de créer un véritable tamis moléculaire.

Selon une version préférée de l'invention, la composition renferme en outre un silicate d'alumine potassique anamorphosé, dont la structure comporte des microcavités.

Ces microcavités ont une dimension comprise entre 1 et 10 microns, la dimension moyenne étant de l'ordre de 5 microns.

Ce composé peut échanger son potassium avec la plupart des cations en solution dans l'eau. La réaction d'échange par exemple avec le plomb sous forme de nitrate, s'écrit :

$$3\ Al_2O_3,\ 6\ SiO_2,\ 2\ K_2O,\ 2\ H_2O + 2\ Pb(NO_3)2 \rightarrow 3\ Al_2O_3\ ,\ 6\ SiO_2,\ 2\ PbO\ ,\ 2\ H_2O + KNO_3$$

Il est évident que comme la plupart des silicates, les alumino-silicates sont des produits totalement insolubles et que le seul moyen de récupérer le métal, une fois fixé, est de solubiliser à l'acide fluorhydrique à chaud et à l'eau régale.

Il a en effet été contrôlé que même en solution acide, les métaux lourds étaient encore captés par le produit ; c'est par exemple le cas dans la fabrication de l'acide phosphorique ; en effet la plupart des phosphates contiennent notamment du cadmium. Ce cadmium est bloqué par le produit selon l'invention à des pH proches de O.

De préférence, les bactéries adsorbées dans lesdites matières minérales, sous forme granulaire sont choisies dans le groupe comprenant les pseudomonas, les bacillus, les lactobacilles et les serratia.

Les bactéries préférées, selon l'invention, sont le bacillus cereus et le bacillus subtilis.

Bien entendu, il est préférable d'utiliser un mélange de bactéries pour augmenter le spectre d'action vis-à-vis des matières organiques contenues dans le milieu pollué.

Les matières minérales utilisées dans la composition selon l'invention, ont une granulométrie préférentielle comprise entre 10 µm et 10 mm.

De préférence également, les proportions des trois matières minérales sont sensiblement égales. Toutefois, on peut privilégier la proportion de l'une ou l'autre matière, en fonction de la nature des polluants qui dominent dans le milieu à épurer.

Par ailleurs, lesdites matières minérales renferment de préférence, à l'état adsorbé, entre environ $10^3$ et $10^{10}$ bactéries par gramme.

Selon un autre aspect de l'invention, le procédé pour la préparation de la composition de matière conforme à l'invention comprend les étapes suivantes :

- on inocule à une température comprise entre 25 et 37°C les bactéries sur un milieu de culture approprié,
- on soumet le milieu de culture inoculé à une ultracentrifugation, de façon à concentrer le milieu en bactéries,
- on disperse le milieu concentré en bactéries sur le mélange desdites matières minérales sous forme granulaire, et
- on sèche l'ensemble à une température inférieure à 40°C.

La durée de l'inoculation (première étape) est par exemple comprise entre 30 et 40 heures.

On va maintenant détailler quelques applications préférées de l'invention.

## TRAITEMENT DES DEVERSEMENTS ACCIDENTELS ET TRAITEMENT DES BOUES

Les déversements accidentels peuvent être une véritable catastrophe pour l'environnement.

Les rejets, les boues et déchets semi-solides, sont maintenant soumis à une réglementation stricte et même souvent interdits en décharge.

La mise en décharge d'un sol contaminé, même autorisé, ne fait que déplacer la pollution.

La mise en oeuvre de la composition selon l'invention apporte une solution efficace au problème des rejets toxiques.

L'association des trois matières de la composition selon l'invention fait fonction de tampon, de stimulateur de croissance, et accélère le processus de dégradation. Pour les pollutions importantes et spécifiques, une addition tous les 8/10 jours de bactéries spécialisées sera faite à la dose appropriée en fonction de la pollution.

L'humidité de déchets, boues ou terres devra être contrôlée scientifiquement afin d'optimiser l'activité biologique.

LISTE DES TOXIQUES POUVANT ETRE DEGRADES ASSEZ FACILEMENT

Phénol - crésol - hydrocarbures halogènes - hydrocarbures naphtaléniques - benzène - triméthylamine - éthanol - chlore-éthane - biphényl - chlorophénol - triglycéride - cellulose - lignine - détergents (tensio-actifs - non ionique - cationiques - anioniques) - certaines graisses et huiles industrielles - certains insecticides herbicides - pepticides (organo phosphorés - organo chlorés) - soufre et composés soufres - dérivés cyanurés.

LISTE DES METAUX LOURDS POUVANT ETRE FIXES

| COBALT | ARSENIC | CADMIUM | PLOMB |
|---|---|---|---|
| CUIVRE | CHROME | Fe++ | Fe+++ |
| ANTIMOINE | ARGENT | CESIUM | STRONTIUM 90 |
| URANIUM | VANADIUM | MERCURE | NICKEL |
| ZINC | TITANE | ETAIN | OR |

Les autres propriétés de la composition selon l'invention, sont les suivantes :

- Rôle équilibrant pour tous déchets végétaux. Elle peut être utilisée pour le compostage, la réactivation biologique des terres pauvres, et le blocage des métaux lourds.

Elle favorise l'assimilation de l'azote par les végétaux.

- Garde une humidité au sol.
- Joue le rôle d'amendement calcaire et magnésium.

Elle peut être également utilisé pour les eaux de consommation avant traitement à l'usine.

Elle supprime les algues microscopiques et relève le pH des eaux acides. Rééquilibre les oligo-éléments et le fluor, et peut être utilisée en pisciculture industrielle comme filtre biologique.

La composition selon l'invention permet également d'éliminer les éléments radioactifs de l'eau.

D'excellents résultats ont ainsi été obtenus sur le césium 137 et strontium 90.

On donne ci-après la description de quelques essais effectués en laboratoire.

Dans ces essais, la mise en oeuvre de la composition préférée selon l'invention, sera appelée "biofixation".

Ces essais avaient pour but de déterminer, à partir d'une eau polluée, et de vase silico-calcique de fond, l'efficacité d'un traitement par bactéries fixées sur différents supports.

L'échantillon de base étant un mélange d'eau de la Marne avec ses vases de fond prélevées sur le lieu dit : "bras du chapitre". Avant le début des essais, de l'azote et du phosphore ont été additionnés à raison de 10 mg/l et 5 mg/l pour 100 mg/l de carbone, de façon à pouvoir juger de l'efficacité du traitement de ces paramètres.

I) Mode opératoire

Dans des éprouvettes en verre de 1 L identiques, le mélange a été introduit après homogénéisation.
Différents supports ont ensuite été utilisés comme l'indique le tableau ci-dessous :

| Tube témoin | 1 | 2 | 3 | 4 | 5 | 6 | 7 |
|---|---|---|---|---|---|---|---|
| Sulfate de calcium | - | 20 | - | - | 20 | - | - |
| Carbonate de chaux | - | - | 20 | - | - | 20 | - |
| Algues corolinacées | - | - | - | 20 | - | - | 20 |
| Biofixation | - | - | - | - | 10 | 10 | 10 |

Chaque jour, et pendant 21 jours, 5% du volume de chaque éprouvette étaient enlevés et remplacés par l'effluent d'origine remis en suspension avec sa vase. Cette opération ayant pour but de simuler un "courant" dans les éprouvettes et de permettre d'éliminer les cellules mortes, comme dans le milieu naturel. Il est à noter

4

que le cycle de 21 jours choisi est en correspondance avec les cycles biologiques naturels (DBO 21).

II) RESULTATS

|  | témoin | C | PG | GG | C | PG | GG |
|---|---|---|---|---|---|---|---|
|  | 1 | 2 | 3 | 4 | 5 | 6 | 7 |
| DCO | 11 | 17 | 13 | 11 | 197 | 139 | 71 |
| DBO | 5 | 5 | 5 | 5 | 80 | 50 | 25 |
| MeS | 23 | 9 | 17 | 11 | 28 | 9 | 11 |
| AZOTE | 9,0 | 3,2 | 4,1 | 4,6 | 2,5 | 2,0 | 10,0 |
| PHOS-PHORES | 34 | 24 | 27 | 21 | 25 | 29 | 26 |
| MS | 53,7 | 46 | 51,6 | 43,5 | 46,6 | 66,9 | 39 |
| rajouts | 53,7 | 26 | 31,6 | 23,5 | 16,6 | 36,9 | 9 |

Signification des symboles :

C : carbonate de chaux          DCO : demande chimique
                                       d'oxygène

PG : petits grains (< 160 µm)   DBO : demande biologique
                                       d'oxygène

GG : gros grains (2 à 10 mm)    MeS : matière en suspension

                                MS  : matière sèche

III/ Interprétation des résultats

1°) Réduction des vases :

Le suivi du phénomène peut se faire sur la chute en matières sèches au fond des tubes.

En effet, par rapport à l'échantillon type (tube n° 1), tous les autres relevés sont nettement inférieurs (après avoir enlevé la partie ajoutée).

On peut considérer que les meilleurs résultats sont obtenus dans l'ordre par :

1) biofixation
2) sulfate de calcium + biofixation
3) algue corolinacée
4) carbonate de chaux

Ces résultats permettent de conclure qu'au niveau de la réduction de la vase, le carbonate de chaux gros grain est le meilleur, suivi du sulfate de calcium, et que la biofixation dans tous les cas améliore nettement le résultat.

Les réductions observées vont de 30 à 83% de la masse de vase du départ par minéralisation des matières organiques.

2°) Eau

Bien que dans l'ensemble des résultats, les matières en suspension soient réduites, il peut paraître étonnant de trouver une augmentation de la DCO et de la DBO.

Etant donné la chute des MES, l'on peut d'ores et déjà en conclure qu'il s'agit de DCO solubles.

L'explication de la montée de la DCO correspond à la minéralisation des vases.

En effet sur une période de 21 jours, la biomasse a le temps de dégrader la plus grosse partie de la matière organique, mais laisse des molécules (acides aminés et autres) non encore dégradés. Etant donné que l'alimentation en effluent était constante et importante (5% par jour), le temps de séjour dans le tube devenait trop court, pour dégrader à la fois la matière organique des vases et la DCO soluble.

De plus, il n'est pas négligeable de considérer la DCO apportée par les bactéries, qui n'est pas une solution mais de la matière vivante entrant dans le cycle naturel.

Pour les DBO, l'augmentation dans le surnageant est plus faible : 2 à 2,5 fois moins, ce qui correspond également à la corrélation classique entre DBO et DCO.

Si l'on considère ces paramètres, il apparaît que l'ordre des résultats pour DCO et DBO montre que le carbonate de chaux gros grain est supérieur au petit grain et au gypse (dans l'ordre). Il est certain qu'un temps supérieur à 21 jours dans des conditions naturelles d'alimentation aurait conduit à une réduction de la DCO et DBO soluble par dégradation biologique.

3°) Azote et phosphore

L'azote est diminué dans les échantillons traités avec des taux de réduction allant de 50 à 78%.

Les meilleurs résultats obtenus sont dans l'ordre:
- BIOFIXATION gros grains, BIOFIXATION petits grains, sulfate de calcium, avec amélioration très nette par la BIOFIXATION.
- De même que pour l'azote, le phosphore soluble est largement diminué par ces traitements entre 14% et 40%.

Les performances enregistrées sont comme suit :
      - Gros grains, petits grains, sulfate de calcium, mais sans effet biologique.

Ce résultat est logique puisque la réduction du phosphore soluble est due à une floculation physico-chimique des phosphates dans laquelle la biologie n'intervient pas ou peu.

Sur l'ensemble des résultats, l'on peut observer une nette diminution des vases, de l'azote et du phosphore.

Les meilleurs traitements obtenus ont été réalisés avec la biofixation et il semble que le carbonate de chaux gros grain devance largement le petit grain et le sulfate de calcium qui sont efficaces sur des points différents.

Dans tous les cas, surtout avec l'addition de bactéries fixées, l'effet du traitement est très visible et devrait l'être encore plus sur site naturel.

DIFFERENTES MESURES COMPARATIVES

Lors d'autres expériences menées en laboratoire, les résultats suivants ont été obtenus :

1) Produit en surface, un an après application

|  | témoin | + produit |
|---|---|---|
| pH | 8,3 | 8,1 |
| oxygène dissous mg/l | 6,2 | 8,1 |

2) Produit mélangé à la vase, moyenne d'une année de mesures

|  | Témoin bac non aéré | + CaCO3 bac non aéré | Témoin bac aéré | + CaCO3 bac aéré |
|---|---|---|---|---|
| oxygène dissous mg/1 | 6,12 | 7,55 | 7,35 | 8,00 |

Au vu de ces résultats, nous constatons que malgré l'application de la BIOFIXATION, le pH reste inférieur à celui du témoin. Cette autorégulation s'explique par la porosité du produit qui stimule l'activité bactérienne (voir expérience 1), élevant ainsi la teneur en acides organiques produit par le métabolisme bactérien.

Par ailleurs, une nette élévation de la teneur en oxygène a lieu. Dans une première phase, c'est la porosité des matériaux employés qui rétablit les conditions aérobies et dans une seconde phase le développement d'une microflore photosynthétique augmente la teneur en oxygène de l'eau.

Des expérimentations ont également été effectuées sur "site".

Une expérience "sur site" a permis de faire les mesures suivantes :

Un an après application :

|  | Témoin | Produit |
|---|---|---|
| pH | 7 | 6,8 |
| oxygène dissous mg/1 | 8 | 11,0 |

Malgré le brassage qui se fait dans l'étang, et par lequel les différences sont atténuées, les résultats mènent aux mêmes conclusions et constatations que celles en laboratoire, c'est-à-dire que le pH varie peu, alors que l'eau s'enrichit en oxygène.

Une expérience a été effectuée "in situ" dans un étang. Cet étang eutrophisé subissait principalement deux sources de pollution : les eaux d'agriculture (matières fertilisantes) et d'un village à proximité de l'étang. La dose de BIOFIXATION répandue a été de 3,5 tonnes à l'hectare.

Le but de ce traitement était d'éliminer l'eutrophisation et de rendre cette eau apte à la pisciculture.
- LA VISIBILITE : après traitement d'automne, les "fleurs d'eau" paraissent après 3 mois même durant l'été favorable à de tels développements.
- L'OXYGENE : l'eau se réoxygène. La stratification en juillet est inverse à une stratification estivale normale. En effet, généralement le fond tend à s'appauvrir en $O_2$ sous l'effet des bactéries hétérotrophes qui décomposent la matière organique. Malgré la masse phytoplanctonique qui s'est déposée sur le fond de l'étang, des conditions aérobies se sont rétablies grâce à l'effet favorable du produit à côté d'une flore hétérotrophe. Par ailleurs, la chaleur particulièrement élevée du mois de juillet tend à réduire l'$O_2$ en surface.
- LE pH : malgré l'apport de $CaCO_3$, la diminution du pH s'explique une fois de plus par l'activité bactérienne, notamment nitrifiante.
- OXYDABILITE AU $KMO_4$ : cette mesure permet d'évaluer le taux en matières organiques dans l'eau. On constate une diminution de celui-ci après traitement.
- L'AZOTE AMMONIACAL $NH_4$, NITREUX $NO_2$, NITRIQUE $NO_3$ ET ORGANIQUE : le taux en nitrates, et

7

dans une moindre mesure en nitrites, s'élève au dépens de l'azote ammoniacal et de l'azote organique. Après traitement, l'ammonification de la matière organique s'accélère, mais cette réaction se poursuit par l'oxydation du $NH_4$ en nitrates. Cette nitrification n'est possible que dans des conditions biologiques plus favorables, telle l'aération du fond.

- PHOSPHORE : l'augmentation soudaine du phosphore constatée en juillet, correspond à l'arrivée des estivants. Cette nouvelle source de pollution (détergents, etc.) est probablement la cause de ce phénomène.

ETUDE PHYTO ET ZOOPLANCTONIQUE DE L'EAU

|  | juil | dec | fev | mai | juil |
|---|---|---|---|---|---|
| PHOTOPLANCTON | 62,11 | 74,72 | 86,28 | 2,66 | 0,15 |
| euchlorophycées | 56,61 | 2,53 | 8,64 | 1,14 | 0,14 |
| cyanophycées | 2,68 | 28,41 | 4,08 | 0,01 | 0,00 |
| diatomées | 1,36 | 1,92 | 56,15 | 0,77 | 0,01 |
| autres | 1,46 | 41,48 | 17,41 | 0,76 | 0,00 |
| ZOOPLANCTON | 31,89 | 25,28 | 13,72 | 97,34 | 99,85 |
| S (degré de saprobie) | 2,33 | 3,58 | 2,35 | 1,40 | 1,84 |

EFFETS SUR LA VASE

1) Avec tubes immergés dans un étang truité.

| Population bactérienne | TEMOIN | + PRODUIT |
|---|---|---|
| ammonification | $2,5 \times 10^5$ | $2 \times 10^6$ |
| cellulolyse aérobie | 9 | 95 |
| minéralisation du S org. | $3,5 \times 10^3$ | $9,5 \times 10^4$ |

Ce tableau montre que les populations bactériennes peuplant les vases des deux tubes se sont nettement accrues dans celui contenant le calcaire poreux. Ces mesures effectuées dix mois après application, permettent d'expliquer la diminution en matières organiques (10 à 15%).

2) Avec tubes carrottiers

| Population bactérienne | TEMOIN | PRODUIT |
|---|---|---|
| ammonification | $4,5 \times 10^4$ | $2,5 \times 10^6$ |
| cellulolyse aérobie | 0 | 25 |
| minéralisation du S org. | $2 \times 10^2$ | $4,5 \times 10^4$ |

Lors de cette expérience, des échantillons de vase prélevés dans l'étang ont été transportés au laboratoire dans les tubes carottiers. Par rapport au témoin le traitement multiplie :
- les ammoniaques par 50
- les minéralisateurs par 200
- les cellulolytiques aérobies : à l'infini

Ces résultats expliquent aisément la baisse considérable en matières organiques (60%).

EP 0 410 877 B1

Différents paramètres avant et aprés le traitement.

| | VISIBI-LITE (m) | O$_2$ DISSOUS | | pH | | Ox KMnO$_4$ | | NH$_4$ mg/1 | | NO$_2$ mg/1 | | NO$_3$ mg/1 | | Mat.Org mg M/1 | HPO$_4^-$ mg/1 |
|---|---|---|---|---|---|---|---|---|---|---|---|---|---|---|---|
| | | 0 m | 2 m | Surface | Fond | Surface | Fond | Surface | Fond | Surface | Fond | Surface | Fond | | Surface |
| juillet | 0,32 | 175 | 171,3 | 8,8 | 7,7 | 7,4 | 7,3 | 0,2 | 2,3 | 0,07 | 0,07 | 5,1 | 1,5 | 5,4 | 0,11 |
| octobre | 0,29 | 69,1 | – | 9,8 | 9,0 | 12,9 | 9,9 | 1,9 | 7,2 | 0,23 | 0,14 | 1,27 | 1,5 | 6,15 | 0,25 |
| février | 0,24 | 88,9 | 112 | 9,0 | 8,9 | 8,0 | 8,5 | 1,8 | 3,4 | 0,13 | 0,10 | 5,4 | 6,9 | 5,25 | 0,70 |
| traitement | | | | | | | | | | | | | | | |
| février | 0,29 | 118,4 | 115,6 | 9,2 | 9,0 | 7,9 | 8,4 | 1,1 | 2,8 | 0,29 | 0,08 | 10,0 | 9,6 | 3,5 | 0,19 |
| mai | 1,70 | 79,5 | 76,6 | 7,55 | 8,2 | 6,0 | 6,4 | 0,6 | 1,0 | 0,24 | 0,26 | 7,1 | 7,1 | 1,8 | 0,30 |
| juillet | 1,82 | 65,32 | 173,4 | 7,6 | 7,7 | 7,7 | 7,9 | 0,3 | 0,3 | 0,03 | 0,02 | 7,1 | 6,1 | 1,35 | 2,8 |

L'oxydabilité au KMnO$_4$ est une mesure de la quantité en matière organique dans l'eau et s'exprime en mg O$_2$/L.

EP 0 410 877 B1

EFFETS SUR METAUX LOURDS

Dans diverses pollutions qui dégradent l'environnement, l'incidence des métaux lourds devient de plus en plus inquiétant :
- que ce soit pour les rejets industriels ou urbains,
- que ce soit les percolations des décharges mêmes contrôlées,
- que ce soit les centres d'incinération ou les traitement de compostage.

Viennent ensuite les pollutions et déversements accidentels, la pollution atmosphérique et leurs retombées.

La BIOFIXATION conforme à la présente invention par sa qualité physico-chimique ionique a formé des complexes quasi définitivement insolubles.

ETUDE DE LA NEUTRALISATION

Elément d'appréciation :

| | |
|---|---|
| pH | : mesuré au pH mètre calorimétrique Hellige. |
| | mesuré au pH mètre au 100 cm Tacussel. |
| T.A.C. | : titration à l'acide sulfurique. |
| Pb | : dosé par la méthode néphélométrique de Dienert. |
| $CO_2$ libre | : volumétrie. |

- ANALYSE DE L'EAU TRAITEE ET ESSAI D'AGRESSIVITE SUR TUYAUX DE PLOMB (durée d'essai 16 heures)

| FILTRE | | | EAU BRUTE | | EAU TRAITEE | | TUYAU DE PLOMB | | | | | |
|---|---|---|---|---|---|---|---|---|---|---|---|---|
| DEBIT | DEBIT | HAUT. | PH | T.A.C | PH | T.A.C | N°1 16/4 NOIR | | | N°2 16/4 ETAM | | |
| VOL/H | M3/H | | | CA0 | | | PH | T.A.C | PB | PH | T.A.C | PB |
| | | | | °/°° | | | | | | | | |
| 10 | 5,6 | 1m | 6 | 13 | 7,3 | 64,5 | 7,3 | 66 | 2,2 | 7,3 | 66 | 1,2 |
| 9,66 | 6 | 1m | 6,1 | 13 | 7,35 | 70 | 7,35 | 70,5 | 0,5 | 7,35 | 71 | 0,5 |
| 8,8 | 5 | 1m | 6,1 | 13 | 7,35 | 64 | 7,4 | 68,5 | 0,4 | 7,4 | 65 | 0,35 |
| 5,45 | 2,7 | 0,88 | 6,1 | 13 | 7,45 | 68,5 | 7,4 | 68 | 0,4 | 7,45 | 67,5 | 0,5 |
| 5,82 | 2,88 | 0,88 | 5,9 | 13 | 7,35 | 69 | 7,35 | 69 | 0,4 | 7,3 | 70 | 0,4 |
| 3,63 | 1,8 | 0,88 | 6 | 13 | 7,4 | 67,5 | 7,45 | 69 | 0,35 | 7,4 | 69 | 0,4 |
| 3,27 | 1,62 | 0,88 | 6 | 13 | 7,4 | 67,5 | 7,45 | 68 | 0,5 | 7,4 | 68,5 | 0,4 |
| 3,39 | 1,68 | 0,88 | 5,9 | 13 | 7,4 | 67 | 7,45 | 67 | 0,5 | 7,6 | 68 | 0,6 |
| 3,03 | 1,5 | 0,88 | 6 | 13 | 7,45 | 66,5 | 7,5 | 67 | 0,3 | 7,5 | 66,5 | 0,8 |
| 3,15 | 1,56 | 0,88 | 5,9 | 13 | 7,4 | 64,5 | 7,4 | 64 | 0,45 | 7,4 | 64 | 0,9 |
| 3,15 | 1,56 | 0,88 | 5,9 | 13 | 7,4 | 65 | 7,45 | 63 | 0,25 | 7,4 | 64 | 0,6 |

11

- <u>RESULTATS DE SERIES D'ESSAIS CONCERNANT LA NEUTRALISATION DE L'EAU</u> (extraits des résultats)

```
 - Granulométrie grosse (3m/m) :            Après 15 h
                                              AV :    AP :
   Débit 5 vol/h                   pH :       5,9      6,3
                                  TAC :       7,6     14,2


 - Granulométrie moyenne (2m/m) :
                                   pH :       5,9      7,1
   Débit 5 vol/h                  TAC :       9,0     20,4
                           CO₂ libre :       48,4     18,5


 - Granulométrie fine, diamètre 1 m/m :
   Essai pH variable               pH :       6,1      7,0
                                  TAC :      10,8     22,8
                           CO₂ libre :       57,0     26,0
        -----------------------------------------
                                   pH :       6,3      7,1
                                  TAC :      11,4     21,6
                           CO₂ libre :       50,0     24,0
        -----------------------------------------
                                   pH :       6,7      7,1
                                  TAC :      15,6     20,4
                           CO₂ libre :       27,0     15,0
```

ETUDE DE L'ELIMINATION DES ELEMENTS TOXIQUES OU INDESIRABLES DE L'EAU :

Il est possible de se trouver en présence d'une eau contenant des éléments toxiques ou indésirables (plomb, cuivre, fer, manganese, chlore, uranium, fluor, arsenic, etc.).
Pour s'en débarrasser, on peut faire appel à :

- L'ABSORPTION CHROMATOGRAPHIQUE

La chromatographie par absorption s'emploiera sans grande modification ; il faudra, bien entendu, que les absorbants soient polaires, ce qui nous permet de dire que cette chromatographie est déjà un échange d'ions.
Le carbonate de sodium, de baryum, de magnésium, de zinc et surtout de calcium ont été utilisés comme absorbants.
La composition de l'ensemble principalement formé de $CaO_3$ convient parfaitement à l'absorption chromatographique. D'ailleurs, si nous faisons passer sur une colonne du produit une solution aqueuse de nitrate de plomb $10^{-2}$M, l'eau se trouve débarrassée totalement des ions Pb. La retenue est aussi spectaculaire avec les ions cuivriques.
Le carbonate de calcium agit à la façon de l'alumine. En présence d'eau, on peut écrire :
$$BASE + H_2O \rightleftarrows ACIDE + HO^-$$
Ces ions $HO^-$ donneront, avec les ions métalliques, des sels basiques ou des oxydes qui précipiteront.
On peut donc, avec intérêt, étudier parallèlement la séparation des ions par précipitation à l'état d'hydroxy-

12

des.

ECHANGES D'IONS :

- ETUDE COMPARATIVE DE LA PRECIPITATION DES CATIONS DANS LES SOLUTIONS AQUEUSES PAR LE CARBONATE DE CALCIUM ET LE SILICATE DE CALCIUM.

Nous avons voulu rechercher la raison des différences de précipitations par le carbonate de calcium et par le silicate de calcium.

| Eau en contact avec: | pH exp. à 20° | pH calculé | T H | Résistivité en ohms |
|---|---|---|---|---|
| 1) le carbonate de calcium | 10,06 | 10,06 | 2,6 | 25.670 |
| 2) procédé de BIOFIXATION | 9,99 | – | 3,5 | 6.040 |
| 3) le silicate de calcium | 10,86 | 11,14 | 6,4 | 996 |

Etant donné les résultats de précipitation des hydroxydes et des sels basiques obtenus, on peut conclure que ce n'est pas le carbonate de calcium qui fixe le pH de précipitation, mais le carbonate acide de calcium, et ce sera les sels basiques et les hydroxydes précipitant à un pH inférieur à 8,3 qui se trouveront éliminés, ce qui explique la précipitation parfaite, par exemple des ions $FE^{+++}$, $Cu^{++}$, $Al^{+++}$, $Cr^{+++}$, $Zn^{++}$, $Pb^{++}$ alors que le $Co^{+++}$, le $Ni^{++}$, le $Fe^{++}$, le $Mn^{++}$, ne se trouvent pas précipités, par contre ces derniers sont éliminés par action du silicate de calcium hydraté.

Une eau traitée au silicate de calcium et amenée au pH de 10,86, mise dans une conduite en plomb neuf pendant 18 heures, a dissous 1,8 mg de $pb^0/00$.

L'étude du diagramme de solubilité de l'hydrate de $Fe^{+++}$, $Fe^{++}$, et $M^{++}$, fait apparaître que le fer ferreux ne peut être éliminé complètement au pH du bicarbonate de calcium, mais par contre à celui du silicate ainsi que les sels manganeux. Il va de soi que le fer ferrique se trouve dans les deux précipités. Le diagramme de solubilité du $Zn^{++}$, du $Co^{++}$ du Ni++, explique la difficulté de précipitation par le carbonate acide de calcium et sa réalisation par le silicate.

A titre d'exemple, on donne ci-après l'essai comparatif du traitement d'une eau avec la BIOFIXATION et le silicate de calcium hydraté :

EAU BRUTE

| | |
|---|---|
| pH | 6,8 |
| TH | 27°4 |
| Fe | néant |
| $SiO_2$ | 10 mg$^0$/00 |

L'eau brute, après contact de 24 heures sur BIOFIXATION, nous donne :

| | |
|---|---|
| pH | 7,7 |
| TH | 32°6 |

Essai d'agressivité sur plomb : pb néant
Essai d'agressivité sur cuivre: Cu néant
L'eau brute, après contact de 24 heures sur silicate de calcium donne :

| | |
|---|---|
| pH | 10 |
| TH | 14°28 |
| $SiO^2$ | 70 mg$^0$/00 |

Essai d'agressivité sur plomb : Pb 1,8 mg$^0$/00
Essai d'agressivité sur cuivre : Cu O.

- <u>ETUDE DE L'ELIMINATION DES ELEMENTS RADIOACTIFS DE L'EAU</u>

- <u>LES DIFFERENTS ELEMENTS RADIOACTIFS</u> :

Le grand problème mondial est l'élimination des ions radioactifs qui se pose chaque jour.
Les éléments radioactifs contaminants les rejets peuvent être classés en trois catégories :
1) Les radioéléments naturels (thorium, uranium, radium et leurs produits de filiation).
2) Les produits de fission (césium-137) (33 ans) - (strontium-90) (24 ans).
3) Les radioéléments artificiels (extraits des produits de fission).

<u>ESSAIS</u> :

a) <u>Données</u>

On a traité au moyen d'une composition selon l'invention, une eau radioactive en provenance de Saclay et ayant la composition suivante :

| CORPS | POUR 1 LITRE | PERIODE | ENERGIE en MeV | ENERGIE en MeV |
|---|---|---|---|---|
| 137<br>Cs    137<br>Ba | 10 curies | 33 ans<br>20 minutes | 0,51 (92%)<br>1,17 ( 8%) | 0,66 |
| 90<br>Sr    90<br>Y | 14 curies | 20 ans<br>61 heures | 0,61<br>2,3 | |
| 144<br>Cr    144<br>Pr | 62 curies | 282 jours<br><br>17,5 min. | 0,3<br>0,17<br>3,0 | 0,03 à0,13<br>0,2<br>1,2 |
| 106<br>Ru    106<br>Rh | 10 curies | 1 an<br>30 sec. | 0,039<br>3,7 | 0,5 |
| 147<br>Pm | 65 curies | 2,6 ans | 0,22 | |
| 155<br>Eu | 80m.curies | 1,7 ans | 0,17<br>0,24 | 0,014 à<br>0,137 |

Autres sels contenus provenant du traitement chimique à l'état de traces : Al, Ni, Cr, Fe, V.

b) <u>Essais et résultats</u> :

Echantillon A :    capsule aluminium (épiradiation)

Echantillon B :     BIOFIXATION (filtration)
Echantillon C :     BIOFIXATION (filtration)
Echantillon D :     carbonate de calcium (agitation magnétique).

## Comptage impulsions/5 mn     Mesures avec échantillons

**A : 16 impulsions/minute**     **74 638/30 secondes**

**B : 15    "     "**     **11 588     "**

**C : 18    "     "**     **940     "**

**D : 17    "     "**     **13 313     "**

Ces résultats nous font apparaître une très bonne retenue des éléments radioactifs par filtration sur BIOFIXATION en poudre.

AUTRES ESSAIS (résultats)

- Par agitation : (1/10e volume BIOFIXATION) + 5% de borates + 5% de silice.

**a) Produit brut grossier : élimination   facteur décontami-nation**

                        **79%**          **5**

**b) Produit brut moyen   :   85%**          **6,7**

- Par percolation :

a) Sur le produit granulé.
Solution mère : 10 577 coups/minute/cc (intégrés).
Soit 0,52. $10^{-2}$ C/cc.

| Vol. en cc | Vol.percolé/Vol.produit | Débit en 1 h: | Coups/min/cc (intégrés) | C/cc | Elimination % | Facteur de décontamination |
|---|---|---|---|---|---|---|
| 100 | 2 | 0,5 | 6 630 | $3.315.10^{-6}$ | 37 | 1,5 |
| 200 | 4 | 0,5 | 8 302 | $4.151.10^{-6}$ | 21 | 1,2 |
| 300 | 6 | 0,5 | 8 948 | $4.474.10^{-6}$ | 15 | 1,2 |
| 400 | 8 | 0,5 | 8 631 | $4.315.10^{-6}$ | 18 | 1,2 |
| 500 | 10 | 0,4 | 9 015 | $4.507.10^{-6}$ | 14 | 1,1 |

b) Sur le produit en poudre grossière.
On opère avec deux solutions mères : la première utilisée jusqu'à 10 volumes, et la deuxième du 10ème au 20ème volume.

1ère solution mère :    26 138 coups/minute/cc (intégrés).
soit 1.17 $10^{-2}$ C/cc

| Vol. en cc | Vol.percolé/Vol.produit | Débit en 1 h | Coups/min/cc (intégrés) | C/cc | Elimination % | Facteur de décontamination |
|---|---|---|---|---|---|---|
| 100 | 2 | 0,9 | 265 | $119.10^{-6}$ | 98,9 | 98,7 |
| 200 | 4 | 0,8 | 320 | $144.10^{-6}$ | 98,7 | 81,2 |
| 300 | 6 | 0,8 | 277 | $124.10^{-6}$ | 98,9 | 94,3 |
| 400 | 8 | 0,7 | 412 | $185.10^{-6}$ | 98,4 | 63,2 |
| 500 | 10 | 1 | 351 | $157.10^{-6}$ | 98,6 | 81,1 |

2ème solution mère :    24 231 coups/minute/cc (intégrés)
soit : 1,09 $10^{-2}$ C/cc

| Vol. en cc | Vol.percolé/Vol.produit | Débit en 1 h | Coups/min/cc (intégrés) | C/cc | Elimination % | Facteur de décontamination |
|---|---|---|---|---|---|---|
| 600 | 12 | 0,6 | 480 | $216.10^{-6}$ | 98 | 50,4 |
| 700 | 14 | 0,6 | 541 | $243.10^{-6}$ | 97,7 | 44,8 |
| 800 | 16 | 0,5 | 701 | $315.10^{-6}$ | 97,1 | 34 |
| 900 | 18 | 0,5 | 849 | $382.10^{-6}$ | 96,5 | 28,5 |
| 1000 | 20 | 0,5 | 1.187 | $543.10^{-6}$ | 95 | 20 |

En matière de décontamination des eaux polluées par radioactivité élevée, un pourcentage d'élimination de 95% doit être considéré comme insuffisant.

Pour rendre potable une eau contenant $10^{-2}$ C/cc, il faut employer un produit qui permet d'obtenir un pourcentage d'élimination de 99,99%, selon les normes actuelles.

On peut penser que ce chiffre pourrait être atteint, compte tenu des résultats obtenus lors de l'élimination des éléments non radioactifs, en faisant 2 traitements :

1) - Traitement par filtration sur BIOFIXATION

2) - Traitement par filtration sur silicate de calcium hydraté, ou même seulement, mise en contact avec ce dernier.

Lors de ce traitement, on peut penser que le STRONTIUM 90 se trouvera pratiquement éliminé, le silicate de calcium opérant un adoucissement.

EP 0 410 877 B1

<u>EFFET SUR L'EPURATION DE L'AIR</u>

Les matières constituant la composition selon l'invention présentent une grande surface de contact et des cavités reliées entre elles par des canaux à dimensions variables entre 2 et 76 angströms représentant de 35 à 50% de leur volume et un minimum de surface de 400 m² au cm³ de matériaux.

Cette structure, sur le plan électrochimique, comprend un électron en plus qui, pour se rééquilibrer, va fixer les ions électro-positifs.

Ceci explique les excellentes propriétés d'échange d'ions. Ces matériaux sont capables d'absorber les molécules gazeuses jusqu'à 30% de leur poids sec pourvu qu'elles soient assez fines pour pénétrer à l'intérieur de ceux-ci.

La distribution des charges électriques dans ce tamis moléculaire donnera une sélectivité dans l'absorption des gaz.

Ainsi le $CO_2$, molécule électrique assymétrique, sera fixé en premier et préférentiellement au méthane (molécule électrique sysmétrique). L'azote ($N_2$) également, sera retenu avant l'oxygène ($O_2$). L'$H_2$ et le $CO_2$ seront donc captés sans problèmes.

Un passage de l'air sous pression ne peut qu'améliorer les mécanismes électriques d'absorption.

On peut dire ainsi que les propriétés physiques de la composition de biofixation selon l'invention permettent à celle-ci d'être un excellent échangeur d'ions naturel (cavité d'échange de 3 à 4 meq/gramme).

Concernant la fixation des bactéries et leur rôle en épuration de l'air, l'efficacité sera liée à l'humidité absorbée et à la matière organique fixée.

La structure microscopique des matériaux offre un ensemble de logements favorables à leur fixation et à leur prolifération.

L'activité bactérienne devrait fournir une décolmatation importante en système continu, évitant ainsi les ennuis de nettoyage répétitif.

Le procédé selon l'invention apporte ainsi une meilleure qualité de l'air épuré avec une servitude réduite par rapport aux autres procédés existants.

**Revendications**

1.  Composition de matière pour l'épuration chimique et biologique des eaux polluées, cette composition étant destinée à être répandue dans l'eau à épurer et étant caractérisée en ce qu'elle comprend au moins les deux matières minérales suivantes, sous forme granulaire :
    - un carbonate de calcium poreux, riche en oligo-éléments,
    - un silicate d'alumine hydraté d'origine zéolitique renfermant des métaux alcalino-terreux, et présentant des microcanaux,
    ces deux matières renfermant à l'état adsorbé des bactéries spécifiques pour la dégradation biologique des matières organiques à chaîne carbonée.

2.  Composition conforme à la revendication 1, caractérisée en ce que le carbonate de calcium est du lithothamnium calcareum.

3.  Composition conforme à l'une des revendications 1 ou 2, caractérisée en ce qu'elle renferme en outre un silicate d'alumine potassique anamorphosé, dont la structure comporte des micro-cavités.

4.  Composition conforme à l'une des revendications 1 à 3, caractérisée en ce que les bactéries adsorbées dans lesdites matières minérales, sous forme granulaire sont choisies dans le groupe comprenant les pseudomonas, les bacillus, les lactobacilles et les serratia.

5.  Composition conforme à l'une des revendications 1 à 4, caractérisée en ce que lesdites matières minérales ont une granulométrie comprise entre 10 μm et 10 mm.

6.  Composition conforme à l'une des revendications 4 ou 5, caractérisée en ce que les proportions de trois matières minérales sont sensiblement égales.

7.  Composition conforme à l'une des revendications 1 à 6, caractérisée en ce que lesdites matières minérales renferment à l'état adsorbé entre environ $10^3$ et $10^{10}$ bactéries par gramme.

8.  Procédé pour la préparation de la composition de matière conforme à l'une des revendications 1 à 7, ca-

17

ractérisé par les étapes suivantes :
- on inocule à une température comprise entre 25 et 37°C les bactéries sur un milieu de culture approprié,
- on soumet le milieu de culture inoculé à une ultracentrifugation, de façon à concentrer le milieu concentré en bactéries dans le mélange desdites matières minérales sous forme granulaire, et
- on sèche, l'ensemble à une température inférieure à 40°C.

## Patentansprüche

1. Stoffzusammensetzung für die chemische und biologische Klärung von Abwässern, wobei diese Zusammensetzung im zu klärenden Wasser verteilt wird, dadurch gekennzeichnet, daß sie mindestens die zwei folgenden körnigen Mineralstoffe aufweist:
   - ein an Spurenelementen reiches poröses Kalziumkarbonat,
   - ein wasserreiches Aluminiumsilikat zeolithischen Ursprungs, das Erdalkalimetalle enthält und Mikrokanäle aufweist,
   wobei diese zwei Stoffe Bakterien in adsorbiertem Zustand enthalten, die besonders für den biologischen Abbau von organischen Stoffen mit einer Kohlenstoffkette geeignet sind.

2. Zusammensetzung nach Anspruch 1, dadurch gekennzeichnet, daß das Kalziumkarbonat lithothamnium calcareum ist.

3. Zusammensetzung nach Anspruch 1 oder 2, dadurch gekennzeichnet, daß sie des weiteren ein Anamorphose-Kaliumaluminiumsilikat enthält, dessen Struktur Mikrohohlräume aufweist.

4. Zusammensetzung nach einem der Ansprüche 1 bis 3, dadurch gekennzeichnet, daß die in den körnigen Mineralstoffen adsorbierten Bakterien aus der aus Pseudomonas, Bazillen, Lactobazillen und Serratia bestehenden Gruppe ausgewählt sind.

5. Zusammensetzung nach einem der Ansprüche 1 bis 4, dadurch gekennzeichnet, daß die Mineralstoffe eine Korngröße zwischen 10 Mikrometer und 10 Millimeter haben.

6. Zusammensetzung nach Anspruch 4 oder 5, dadurch gekennzeichnet, daß die Anteile der drei Mineralstoffe im wesentlichen gleich sind.

7. Zusammensetzung nach einem der Anspruche 1 bis 6, dadurch gekennzeichnet, daß die Mineralstoffe ca. 1 000 bis 10 000 000 000 Bakterien pro Gramm in adsorbiertem Zustand enthalten.

8. Verfahren zur Herstellung der Stoffzusammensetzung nach einem der Anprüche 1 bis 7, gekennzeichnet durch die folgenden Schritte:
   - die Bakterien werden bei einer Temperatur zwischen 25 und 37°C auf einen Nährboden geimpft,
   - der geimpfte Nährboden wird ultrazentrifugiert, so daß der mit Bakterien angereicherte Nährboden in dem körnigen Mineralstoffgemisch konzentriert wird, und
   - das Ganze wird bei einer Temperatur unter 40°C getrocknet.

## Claims

1. A composition of substance for chemical and biological purification of polluted water, the composition being adapted to be dispersed in water for purification and being characterised in that it comprises at least the following two mineral substances in granular form:
   - a porous calcium carbonate rich in oligo-elements and
   - a hydrated alumina silicate of zeolite origin containing alkaline earth metals and formed with microchannels,
   the two substances containing adsorbed bacteria specific for biological degradation of organic carbonchain substances.

2. A composition according to claim 1, characterised in that the calcium carbonate is lithothamnium calcareum.

3. A composition according to claim 1 or 2, characterised in that it also contains an anamorphosed potassium alumina silicate formed with micro-cavities.

4. A composition according to any of claims 1 to 3, characterised in that the bacteria adsorbed in the mineral substances in granular form are chosen from the group comprising pseudomonas, bacilli, lactobacilli and serratia.

5. A composition according to any of claims 1 to 4, characterised in that the mineral substances have a particle size between 10 μm and 10 mm.

6. A composition according to claim 4 or 5, characterised in that the proportions of three mineral substances are substantially equal.

7. A composition according to any of claims 1 to 6, characterised in that the mineral substances contain between about $10^3$ and $10^{10}$ adsorbed bacteria per gram.

8. A method of preparing the composition of substance according to any of claims 1 to 7, characterised by the following steps:
   - the bacteria are inoculated at a temperature between 25 and 37°C in a suitable culture medium,
   - the inoculated culture medium is ultra-centrifuged so that the medium concentrated in bacteria is concentrated in the mixture of the aforementioned mineral substances in granular form, and
   - the whole is dried at a temperature below 40°C.